# EUROPEAN PATENT SPECIFICATION

(11) **EP 1 874 924 B1**
(45) Date of publication and mention of the grant of the patent: **22.08.2012**
(21) Application number: 06843684.9
(22) Date of filing: 22.12.2006
(51) Int. Cl.: C12N 9/08, C12G 1/02

(54) **Use of catalase genes for assessing or modifying the sulfite-producing capabiliy of yeasts**
Verwendung von Katalase-Genen, um die Sulfit-Produktionsfähigkeit von Hefe zu bemessen oder zu modifizieren
Utilisation des gènes de catalase por évaluer ou modifier la capacité de production de sulfites dans les levures

(30) Priority: 28.02.2006 JP 2006053898
(43) Date of publication of application: 09.01.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 6188503 (JP); KODAMA, Yukiko, Mishima-gun, Osaka 6188503 (JP); SHIMONAGA, Tomoko, Mishima-gun, Osaka 6188503 (JP); OMURA, Fumihiko, Mishima-gun, Osaka 6188503 (JP)
(74) Representative: Vossius & Partner
(86) International application number: PCT/JP2006/326308
(87) International publication number: WO 2007/099692

(56) References cited:
- WO-A2-2006/024951
- US-A1- 2004 265 862
- DATABASE UniProt [Online] 1 January 1988 (1988-01-01), "Catalase T (EC 1.11.1.6)." XP002426958 retrieved from EBI accession no. UNIPROT:P06115 Database accession no. P06115 -& HARTIG A ET AL: "NUCLEOTIDE SEQUENCE OF THE SACCHAROMYCES-CEREVISIAE CTT-1 GENE AND DEDUCED AMINO ACID SEQUENCE OF YEAST CATALASE T" EUROPEAN JOURNAL OF BIOCHEMISTRY, vol. 160, no. 3, 1986, pages 487-490, XP002426849 ISSN: 0014-2956
- DATABASE UniProt [Online] 16 August 2004 (2004-08-16), "Similar to sp P06115 Saccharomyces cerevisiae YGR088w CTT1 catalase T." XP002426959 retrieved from EBI accession no. UNIPROT:Q6CQS8 Database accession no. Q6CQS8 & DUJON BERNARD ET AL: "Genome evolution in yeasts." NATURE 1 JUL 2004, vol. 430, no. 6995, 1 July 2004 (2004-07-01), pages 35-44, ISSN: 1476-4687
- DATABASE EMBL [Online] 10 January 2001 (2001-01-10), "T3 end of clone AS0AA018G03 of library AS0AA from strain CLIB 533 of Saccharomyces bayanus" XP002426960 retrieved from EBI accession no. EMBL:AL399994 Database accession no. AL399994 -& SOUCIET JEAN-LUC ET AL: "Genomic exploration of the hemiascomycetous yeasts: 1. A set of yeast species for molecular evolution studies" FEBS LETTERS, vol. 487, no. 1, 22 December 2000 (2000-12-22), pages 3-12, XP004337869 ISSN: 0014-5793 -& BON ELISABETH ET AL: "Genomic exploration of the hemiascomycetous yeasts: 5. Saccharomyces bayanus var. uvarum" FEBS LETTERS, vol. 487, no. 1, 22 December 2000 (2000-12-22), pages 37-41, XP004337873 ISSN: 0014-5793
- DATABASE EMBL [Online] 10 January 2001 (2001-01-10), "T7 end of clone AU0AA010F02 of library AU0AA from strain CBS 3082 of Saccharomyces kluyveri" XP002426961 retrieved from EBI accession no. EMBL:AL406507 Database accession no. AL406507 -& NEUVEGLISE C ET AL: "Genomic Exploration of the Hemiascomycetous Yeasts: 9. Saccharomyces kluyveri" FEBS LETTERS, ELSEVIER, AMSTERDAM, NL, vol. 487, no. 1, 22 December 2000 (2000-12-22), pages 56-60, XP004337877 ISSN: 0014-5793
- KORCH C ET AL: "A MECHANISM FOR SULFITE PRODUCTION IN BEER AND HOW TO INCREASE SULFITE LEVELS BY RECOMBINANT GENETICS" PROCEEDINGS OF THE EUROPEAN BREWERY CONVENTION CONGRESS. LISBON, 1991, OXFORD, OUP, GB, vol. CONGRESS 23, 1991, pages 201-208, XP002066286
- HANSEN J ET AL: "Modification of biochemical pathways in industrial yeasts" JOURNAL OF BIOTECHNOLOGY, ELSEVIER SCIENCE PUBLISHERS, AMSTERDAM, NL, vol. 49, no. 1, 20 August 1996 (1996-08-20), pages 1-12, XP004037090 ISSN: 0168-1656
- GIBSON B.R. ET AL: "Oxygen as toxin: oxidative stress and brewing yeast physiology.", CEREVISIA, vol. 31, no. 1, 31 January 2006 (2006-01-31), pages 25-36, XP009111297, UK ISSN: 0770-1713
- DATABASE EMBL [Online] 05 June 2009 'Sequence 4159 from Patent WO2006024951.' Retrieved from EBI, accession no. EMBL:HB145805 Database accession no. HB145805
- DATABASE EMBL [Online] 05 June 2009 'Sequence 76750 from Patent WO2006024951.' Retrieved from EBI, accession no. EMBL:HB218396 Database accession no. HB218396

### SEQUENCE LISTING

<110> Suntory Limited
<120> Catalase gene and use thereof
<130> PCT06-0137
<150> JP2006-53898
   <151> 2006-02-28
<160> 8
<170> PatentIn version 3.3
<210> 1
   <211> 1683
   <212> DNA
   <213> Saccharomyces sp.
<400> 1
   atgtttggta aaaaggaaga aaagcctgag aaagtttaca ctttgcaaaa tgggttccca 60
   tactctcatc acccatacgc ctcccaatac tcaagacccg acggccctat cttgctgcaa 120
   gacttccatc tattggagaa tatcgctagc tttgacagag aaagagtacc agagcgtgta 180
   gtccacgcca aaggtggtgg ttgtagatta gaattcgaat tgacggattc tttgagcgac 240
   attacatacg ctgctcctta ccaagaagtg ggttacaaat gccccggcct tgtccgtttc 300
   tccactgttg gtggtgaaag tggtaccccg gacactgcaa gagacccaag aggtgtttct 360
   ttcaaattct acactaaatg gggcaaccac gactgggtct ttaataacac acccgtattc 420
   tttttaaggg atgccatcaa gttccctgca ttcattcaca cccaaaagag agacccccaa 480
   agtcacctga atcaattcga ggacactacc ctttactggg attatttgac tatgaacccc 540
   gagtcgattc accaaatcac atatatgttt ggtgagagag gtactcctgc ctcttggtct 600
   gacatgaacg cttactccgg ccactcatat atcatggtca acaaagaggg gaaggacact 660
   tatgtacagt ttcacgtcct gtcggatact ggtttcgaaa cattgaccgg cgacaaagct 720
   gctgaactat ctggttctca tccagactat aaccaggcta agttgttcac acaaatgcaa 780
   aacggtgaaa agccaaaatt cacctgttat gtacaaacaa tgactcctga acaagccgcc 840
   aagttcaaat actcggttaa tgacttgacc aagatctggc cgcacaaaga attcccattg 900
   agaaaattcg gtaccatcac gttgactact aacgttgaca actactttca agaaatcgag 960
   caagtagcat tcagtccaac caacacttgt attccaggta ttaaaccttc caatgattca 1020
   gttctacagg ctagactctt ctcttaccca gacacgcaac gacacagact aggggccaac 1080
   tatcagcaac tgcctgtcaa cagaccaaga aatcttggat gtccatactc taaggaggac 1140
   tcccaatact ctgccgaaca gtgtccattc aaggctgcta actttcaaag agacggccca 1200
   atgagttact ataactttgg tcctgaacca aactatctct ccagtttacc aaaccaaact 1260
   ctcaaattca aaaatgaaca aaacgatgaa gtgtctgacg aattcaaagg aatagttcta 1320
   gatgaagtta ccaaggcttc tttgaaaaaa caagaagaag accaaatcag aaatgaacat 1380
   atcgtcgatg ctaagatcaa tcaatattat tacgtctacg gtatgagtcc ccttgacttc 1440
   gaacaaccta gagctttatt tgaaaaagtt tacgaccctg aacaaaagaa gctattcgtt 1500
   cataacgttg tttgccatgc ttgtaagatt aaagatccta atgtgaaaaa gagagttacc 1560
   caatactttg gtttgttgaa cgaggacttg ggtaaagtca tcgctgaggg cctggaggtt 1620
   gaatgggaac ctgtcgacct tcaaggttac gccaagactt ggagcattgc aagtgcaaac 1680
   taa 1683
<210> 2
   <211> 560
   <212> PRT
   <213> Saccharomyces sp.
<400> 2
   Met Phe Gly Lys Lys Glu Glu Lys Pro Glu Lys Val Tyr Thr Leu Gln
   1               5                   10                  15
   Asn Gly Phe Pro Tyr Ser His His Pro Tyr Ala Ser Gln Tyr Ser Arg
               20                  25                  30
   Pro Asp Gly Pro Ile Leu Leu Gln Asp Phe His Leu Leu Glu Asn Ile
           35                  40                  45
   Ala Ser Phe Asp Arg Glu Arg Val Pro Glu Arg Val Val His Ala Lys
       50                  55                  60
   Gly Gly Gly Cys Arg Leu Glu Phe Glu Leu Thr Asp Ser Leu Ser Asp
   65                  70                  75                  80
   Ile Thr Tyr Ala Ala Pro Tyr Gln Glu Val Gly Tyr Lys Cys Pro Gly
                   85                  90                  95
   Leu Val Arg Phe Ser Thr Val Gly Gly Glu Ser Gly Thr Pro Asp Thr
               100                 105                 110
   Ala Arg Asp Pro Arg Gly Val Ser Phe Lys Phe Tyr Thr Lys Trp Gly
           115                 120                 125
   Asn His Asp Trp Val Phe Asn Asn Thr Pro Val Phe Phe Leu Arg Asp
       130                 135                 140
   Ala Ile Lys Phe Pro Ala Phe Ile His Thr Gln Lys Arg Asp Pro Gln
   145                 150                 155                 160
   Ser His Leu Asn Gln Phe Glu Asp Thr Thr Leu Tyr Trp Asp Tyr Leu
   165 170 175
   Thr Met Asn Pro Glu Ser Ile His Gln Ile Thr Tyr Met Phe Gly Glu
   180 185 190
   Arg Gly Thr Pro Ala Ser Trp Ser Asp Met Asn Ala Tyr Ser Gly His
   195 200 205
   Ser Tyr Ile Met Val Asn Lys Glu Gly Lys Asp Thr Tyr Val Gln Phe
   210 215 220
   His Val Leu Ser Asp Thr Gly Phe Glu Thr Leu Thr Gly Asp Lys Ala
   225 230 235 240
   Ala Glu Leu Ser Gly Ser His Pro Asp Tyr Asn Gln Ala Lys Leu Phe
   245 250 255
   Thr Gln Met Gln Asn Gly Glu Lys Pro Lys Phe Thr Cys Tyr Val Gln
   260 265 270
   Thr Met Thr Pro Glu Gln Ala Ala Lys Phe Lys Tyr Ser Val Asn Asp
   275 280 285
   Leu Thr Lys Ile Trp Pro His Lys Glu Phe Pro Leu Arg Lys Phe Gly
   290 295 300
   Thr Ile Thr Leu Thr Thr Asn Val Asp Asn Tyr Phe Gln Glu Ile Glu
   305 310 315 320
   Gln Val Ala Phe Ser Pro Thr Asn Thr Cys Ile Pro Gly Ile Lys Pro
   325 330 335
   Ser Asn Asp Ser Val Leu Gln Ala Arg Leu Phe Ser Tyr Pro Asp Thr
   340 345 350
   Gln Arg His Arg Leu Gly Ala Asn Tyr Gln Gln Leu Pro Val Asn Arg
   355 360 365
   Pro Arg Asn Leu Gly Cys Pro Tyr Ser Lys Glu Asp Ser Gln Tyr Ser
   370 375 380
   Ala Glu Gln Cys Pro Phe Lys Ala Ala Asn Phe Gln Arg Asp Gly Pro
   385 390 395 400
   Met Ser Tyr Tyr Asn Phe Gly Pro Glu Pro Asn Tyr Leu Ser Ser Leu
   405 410 415
   Pro Asn Gln Thr Leu Lys Phe Lys Asn Glu Gln Asn Asp Glu Val Ser
   420 425 430
   Asp Glu Phe Lys Gly Ile Val Leu Asp Glu Val Thr Lys Ala Ser Leu
   435 440 445
   Lys Lys Gln Glu Glu Asp Gln Ile Arg Asn Glu His Ile Val Asp Ala
   450 455 460
   Lys Ile Asn Gln Tyr Tyr Tyr Val Tyr Gly Met Ser Pro Leu Asp Phe
   465 470 475 480
   Glu Gln Pro Arg Ala Leu Phe Glu Lys Val Tyr Asp Pro Glu Gln Lys
   485 490 495
   Lys Leu Phe Val His Asn Val Val Cys His Ala Cys Lys Ile Lys Asp
   500 505 510
   Pro Asn Val Lys Lys Arg Val Thr Gln Tyr Phe Gly Leu Leu Asn Glu
   515 520 525
   Asp Leu Gly Lys Val Ile Ala Glu Gly Leu Glu Val Glu Trp Glu Pro
   530 535 540
   Val Asp Leu Gln Gly Tyr Ala Lys Thr Trp Ser Ile Ala Ser Ala Asn
   545 550 555 560
<210> 3
   <211> 1689
   <212> DNA
   <213> Saccharomyces sp.
<400> 3
   atgaacgtgt tcggtaaaaa agaagaaaag caagaaaaag tttactctct acaaaacggt 60
   tttccgtact ctcatcaccc atacgcttct caatactcaa gaccagacgg ccctatctta 120
   ctgcaagact tccatctgct ggaaaatatc gcaagtttcg atagagaaag agttccggag 180
   cgtgtagtcc atgccaaagg tggtggttgt agactggagt tcgaactaac agattctttg 240
   agtgatatta catacgccgc tccataccag aatgtgggtt acaaatgtcc tggtcttgtt 300
   cgtttttcca ccgttggtgg tgaaagtggt acaccagaca ctgcaagaga cccaagaggt 360
   gtttctttta aattctatac cgagtggggg aaccatgact gggtcttcaa caatactccc 420
   gtcttcttcc tcagagacgc tattaagttt cccgtattta ttcattcgca aaagagagac 480
   cctcagtctc atctgaatca gtttcaggac actaccatat actgggatta tctaacattg 540
   aatccggaat caatccatca aataacttac atgtttggtg atagaggtac tcctgcttcg 600
   tgggctagta tgaacgcgta ctctggtcat tccttcatca tggtcaacaa agaaggtaag 660
   gacacatatg tgcaattcca cgtcttgtcg gatactggtt ttgaaacctt gactggagat 720
   aaggctgctg aactgtcagg ctcccaccct gattataatc aggcaaagct gttcactcaa 780
   ttgcaaaatg gcgaaaagcc aaaatttaac tgttatgtgc aaacaatgac acccgaacaa 840
   gcaactaagt tcaggtattc ggtaaatgac ctaacgaaaa tatggccaca caaggaattc 900
   cctttgagaa aatttggtac catcacccta acggagaatg ttgacaatta tttccaagaa 960
   attgaacaag ttgcattcag tccaacgaac acttgtatcc caggtattaa gccttctaat 1020
   gattccgttc tacaagccag acttttctcc tatccagaca ctcaacgtca tagattggga 1080
   gccaactatc accaattgcc tgtcaacaga ccaagaaact tgggatgtcc atactccaaa 1140
   ggtgattccc aatacactgc cgaacagtgt ccatttaaag cagtgaactt ccaaagggac 1200
   ggcccaatga gttactacaa tttcggtcct gagccaaatt atatttctag tttaccaaat 1260
   caaactctga aattcaaaaa tgaagacaac aacgaagtat ctgataagtt caaagggata 1320
   gttcttgacg aagtaacaga agtttctgtg agaaagcagg aacaagagca aatcagaaac 1380
   gagcatattg ttgatgccaa aattaatcaa tattactacg tttatggtat tagtccacta 1440
   gacttcgaac agccaagagc tctatatgaa aaggtataca acgatgaaca gaagaaatta 1500
   ttcgttcata acgttgtttg ccacgcttgt aagatcaaag atcctaaagt caaaaagaga 1560
   gttacgcaat actttggttt gctaaacgaa gatttgggta aagtcattgc agaaggcttg 1620
   ggagttcctt gggaacctgt tgaccttgaa ggttatgcca agacttggtc cattgcaagt 1680
   gccaattaa 1689
<210> 4
   <211> 562
   <212> PRT
   <213> Saccharomyces sp.
<400> 4
   Met Asn Val Phe Gly Lys Lys Glu Glu Lys Gln Glu Lys Val Tyr Ser
   1 5 10 15
   Leu Gln Asn Gly Phe Pro Tyr Ser His His Pro Tyr Ala Ser Gln Tyr
   20 25 30
   Ser Arg Pro Asp Gly Pro Ile Leu Leu Gln Asp Phe His Leu Leu Glu
   35 40 45
   Asn Ile Ala Ser Phe Asp Arg Glu Arg Val Pro Glu Arg Val Val His
   50 55 60
   Ala Lys Gly Gly Gly Cys Arg Leu Glu Phe Glu Leu Thr Asp Ser Leu
   65 70 75 80
   Ser Asp Ile Thr Tyr Ala Ala Pro Tyr Gln Asn Val Gly Tyr Lys Cys
   85 90 95
   Pro Gly Leu Val Arg Phe Ser Thr Val Gly Gly Glu Ser Gly Thr Pro
   100 105 110
   Asp Thr Ala Arg Asp Pro Arg Gly Val Ser Phe Lys Phe Tyr Thr Glu
   115 120 125
   Trp Gly Asn His Asp Trp Val Phe Asn Asn Thr Pro Val Phe Phe Leu
   130 135 140
   Arg Asp Ala Ile Lys Phe Pro Val Phe Ile His Ser Gln Lys Arg Asp
   145 150 155 160
   Pro Gln Ser His Leu Asn Gln Phe Gln Asp Thr Thr Ile Tyr Trp Asp
   165 170 175
   Tyr Leu Thr Leu Asn Pro Glu Ser Ile His Gln Ile Thr Tyr Met Phe
   180 185 190
   Gly Asp Arg Gly Thr Pro Ala Ser Trp Ala Ser Met Asn Ala Tyr Ser
   195 200 205
   Gly His Ser Phe Ile Met Val Asn Lys Glu Gly Lys Asp Thr Tyr Val
   210 215 220
   Gln Phe His Val Leu Ser Asp Thr Gly Phe Glu Thr Leu Thr Gly Asp
   225 230 235 240
   Lys Ala Ala Glu Leu Ser Gly Ser His Pro Asp Tyr Asn Gln Ala Lys
   245 250 255
   Leu Phe Thr Gln Leu Gln Asn Gly Glu Lys Pro Lys Phe Asn Cys Tyr
   260 265 270
   Val Gln Thr Met Thr Pro Glu Gln Ala Thr Lys Phe Arg Tyr Ser Val
   275 280 285
   Asn Asp Leu Thr Lys Ile Trp Pro His Lys Glu Phe Pro Leu Arg Lys
   290 295 300
   Phe Gly Thr Ile Thr Leu Thr Glu Asn Val Asp Asn Tyr Phe Gln Glu
   305 310 315 320
   Ile Glu Gln Val Ala Phe Ser Pro Thr Asn Thr Cys Ile Pro Gly Ile
   325 330 335
   Lys Pro Ser Asn Asp Ser Val Leu Gln Ala Arg Leu Phe Ser Tyr Pro
   340 345 350
   Asp Thr Gln Arg His Arg Leu Gly Ala Asn Tyr His Gln Leu Pro Val
   355 360 365
   Asn Arg Pro Arg Asn Leu Gly Cys Pro Tyr Ser Lys Gly Asp Ser Gln
   370 375 380
   Tyr Thr Ala Glu Gln Cys Pro Phe Lys Ala Val Asn Phe Gln Arg Asp
   385 390 395 400
   Gly Pro Met Ser Tyr Tyr Asn Phe Gly Pro Glu Pro Asn Tyr Ile Ser
   405 410 415
   Ser Leu Pro Asn Gln Thr Leu Lys Phe Lys Asn Glu Asp Asn Asn Glu
   420 425 430
   Val Ser Asp Lys Phe Lys Gly Ile Val Leu Asp Glu Val Thr Glu Val
   435 440 445
   Ser Val Arg Lys Gln Glu Gln Glu Gln Ile Arg Asn Glu His Ile Val
   450 455 460
   Asp Ala Lys Ile Asn Gln Tyr Tyr Tyr Val Tyr Gly Ile Ser Pro Leu
   465 470 475 480
   Asp Phe Glu Gln Pro Arg Ala Leu Tyr Glu Lys Val Tyr Asn Asp Glu
   485 490 495
   Gln Lys Lys Leu Phe Val His Asn Val Val Cys His Ala Cys Lys Ile
   500 505 510
   Lys Asp Pro Lys Val Lys Lys Arg Val Thr Gln Tyr Phe Gly Leu Leu
   515 520 525
   Asn Glu Asp Leu Gly Lys Val Ile Ala Glu Gly Leu Gly Val Pro Trp
   530 535 540
   Glu Pro Val Asp Leu Glu Gly Tyr Ala Lys Thr Trp Ser Ile Ala Ser
   545 550 555 560
   Ala Asn
<210> 5
   <211> 40
   <212> DNA
   <213> Artificial
<220>
   <223> Primer
<400> 5
   gagctcatag cggccatgtt tggtaaaaag gaagaaaagc 40
<210> 6
   <211> 42
   <212> DNA
   <213> Artificial
<220>
   <223> Primer
<400> 6
   ggatcctatg cggccgcgta tatatgtaat aaataatagt gc 42
<210> 7
   <211> 21
   <212> DNA
   <213> Artificial Sequence
<220>
   <223> Primer
<400> 7
   gtcgacatga acgtgttcgg t 21
<210> 8
   <211> 23
   <212> DNA
   <213> Artificial Sequence
<220>
   <223> Primer
<400> 8
   gcggccgcaa tagtgctgcc tta 23

## Description

### TECHNICAL FIELD

Disclosed is a gene encoding catalase and use thereof, in particular a brewery yeast for producing alcoholic beverages with superior flavor, alcoholic beverages with increased content of sulfite produced with said yeast, and a method for producing said beverages. More particularly, the invention relates to a yeast, whose capability of producing sulfite that contribute to stability of flavor in products, is enhanced by amplifying expression level of CTT1 gene encoding Cttlp that is a catalase in a brewery yeast, especially non-ScCTT1 gene or ScCTT1 gene specific to a lager brewing yeast, and to a method for producing alcoholic beverages with increased content of sulfite with said yeast.

### BACKGROUND ART

Sulfite has been known as a compound having high anti-oxidative activity, and thus has been widely used in the fields of food, beverages, pharmaceutical products or the like (for example, Japanese Patent Application Laid-Open Nos. H06-040907 and 2000-093096). In alcoholic beverages, sulfite has been used as an anti-oxidant. For example, because sulfite plays an important role in quality maintenance of wine that needs long-term maturation, addition of up to 350 ppm (parts per million) of residual concentration is permitted by the Ministry of Health, Welfare and Labor in Japan. Further, it is also know that shelf life (quality maintained period) varies depending upon sulfite concentration in a product in beer brewing. Thus, it is quite important to increase the content of this compound from the viewpoint of flavor stability or the like.

The easiest way to increase the sulfite content in a product is addition of sulfite. However, sulfite is treated as a food additive, resulting in some problems such as constraint of product development and negative images of food additives of consumers.

Methods of increasing sulfite content in a fermentation liquor during brewing process include (1) a method based on process control, and (2) a method based on breeding of yeast. In the method based on a process control, since the amount of sulfite produced is in inverse proportion to the amount of initial oxygen supply, supplied amount of oxygen is reduced to increase amount of sulfite produced and to prevent oxidation.

On the other hand, gene manipulation techniques are used in the method based on breeding of yeast. Yeast biosynthesizes sulfur-containing compounds which are required for its biological activity. Sulfite is produced as an intermediate in the biosynthesis of sulfur-containing compounds. Thus, amount of sulfite in products can be increased by utilizing the ability of yeast without adding sulfite from outside.

The MET3 and MET14 are genes encoding reductases participating in steps which are involved in biosynthesis of sulfite from sulfate ion taken from culture medium. Korch et al. attempted to increase a sulfite-producing capability of yeasts by increasing expression level of the two genes, and found that MET14 is more effective (C. Korch et al., Proc. Eur. Brew. Conv. Conger., Lisbon, 201-208, 1991). Also, Hansen et al attempted to increase production amount of sulfite by disrupting MET10 gene encoding a sulfite ion reductase to prevent reduction of sulfite produced (J. Hansen et al., Nature Biotech., 1587-1591, 1996). On the other hand, however, delay in fermentation or increase in acetaldehyde and 1-propanol, which are undesirable flavor ingredients, are also observed.

Further, Fujimura et al. attempted to increase sulfite content in beer by increasing expression level of a non-ScSSU1 gene unique to a lager brewing yeast among SSU1 genes encoding sulfite ion efflux pump of yeast to promote excretion of sulfite to outside the yeast cells (Fujimura et al., Abstract of 2003 Annual Conference of the Japan Society for Bioscience, Biotechnology and Agrochem., 159, 2003). The patent application US2004/265862 disclosed methods for selecting genes from industrial yeasts, said genes contributing to a desired characteristic for yeasts used for producing alcoholic beverages, in particular to genes which increase sulfite production in said yeasts during alcoholic fermentation. Several genes increasing sulfite production were identified, including SSU1, MET14, and others. The alcoholic beverages produced had an increased sulfite content. In addition, several catalases were known in yeasts, including catalase T from *S. cervisiae* and other yeast species. Hartig A. et al., Eur. J. Biochem. 160: 487-490 (1986) and the related database entry UNIPROT:P06115 "Catalase T (EC 1.11.1.6).", disclose the CTT-1 gene from *S. cerevisiae*, coding for Catalase T, with 93.04 % identity over 560 amino acids with SEQ ID No: 2 of the application and 99.29 % identity over 562 amino acids with SEQ ID NO: 4. Dujon Bernard et al., Nature 430: 35-44 (2004) and the referred database entry UNIPROT: Q6CQS8 disclose a protein from *Kluyveromyces lactis* similar to catalase CTT1 from *S. cerevisiae.* Bon Elisabeth et al., FEBS Letters 487: 37-41 (2000) and the database EMBL: AL399994 discloses a sequence from *Saccharomyces uvarum* similar to *Saccharomyces cerevisiae* ORF YGR088w, coding for CTT1 (catalase T). Neuveglise et al., FEBS letters 487: 56-60 (2000) and EMBL:AL406507 disclose a sequence from *Saccharomyces kluyveri (Lachancea kluyveri),* similar to *Saccharomyces cerevisiae* ORF YGR088w, coding for CTT1 (catalase T, cytosolic). However, there was no disclosure that said catalases were related to sulfite-production in yeasts.

### DISCLOSURE OF INVENTION

As mentioned above, the easiest way to increase sulfite content in a product is addition of sulfite. However, it is desirable to minimize use of food additives in view of recent consumers' preference, i.e., avoidance of food additives and preference of natural materials. Thus, it is desirable to achieve sulfite content which is effective level for flavor stability by use of biological activity of yeast itself without adding sulfite from outside. However, the method based on a process control as described above may not be practical since shortage of oxygen may cause decrease in growth rate of yeasts, resulting in delay in fermentation and quality loss.

Further, in breeding of yeast using gene manipulation techniques, there is a report stating that ten times or more sulfite content than parent strain was achieved (J. Hansen et al., Nature Biotech., 1587-1591, 1996). However, there are problems such as delay in fermentation and increase of undesirable flavor ingredients such as acetaldehyde and 1-propanol. Thus, there are problems with the yeast for practical use. Thus, there has been a need for a method for breeding yeast capable of producing sufficient amount of sulfite without impairing the fermentation rates and quality of the products.

To solve the problems described above, the present inventors made extensive studies, and as a result succeeded in identifying and isolating a yeast in which a gene encoding a catalase from a lager brewing yeast was transformed and expressed was produced to confirm increase in production of sulfite, thereby completing the present invention.

Thus, herein disclosed is a catalase gene existing in a lager brewing yeast, to a protein encoded by said gene, to a transformed yeast in which the expression of said gene is controlled, to a method for controlling the amount of sulfite produced in a product by using a yeast in which the expression of said gene is controlled. More specifically, the present invention relates to the following items:
[1] A method for assessing a test yeast for its sulfite-producing capability, comprising:
   (a) culturing a test yeast; and
   (b) measuring the expression level of a gene encoding a protein having catalase activity said gene having the nucleotide sequence of SEQ ID NO: 1 or SEQ ID NO: 3 by using a primer or a probe designed based on the nucleotide sequence of SEQ ID NO: 1 or SEQ ID NO: 3.
[2] A method for selecting a yeast, comprising:
   (a) culturing a test yeast;,
   (b) quantifying the protein encoded by the polynucleotide selected from the group consisting of:
      (i) a polynucleotide comprising the nucleotide sequence of SEQ ID NO:1;
      (ii) a polynucleotide encoding a protein consisting of the amino acid sequence of SEQ ID NO:2; and
      (iii) a polynucleotide encoding a protein having an amino acid sequence having 93% or higher identity with the amino acid sequence of SEQ ID NO:2 having catalase activity and an enhanced sulfite-producing capability in yeast or measuring the expression level of a gene encoding a protein having catalase activity said gene having the nucleotide sequence of SEQ ID NO: 1 or SEQ ID NO: 3; and
   (c) selecting a test yeast having desired amount of said protein or desired expression level of said gene in view of a desired sulfite-producing capability.
[3] The method for selecting a yeast according to [2], comprising:
   (a) culturing a reference yeast and a test yeast,
   (b) measuring the expression level of a gene encoding a protein having catalase activity in each yeast, said gene having the nucleotide sequence of SEQ ID NO: 1 or SEQ ID NO: 3; and
   (c) selecting a test yeast having the gene expressed higher than that in the reference yeast.
[4] The method for selecting a yeast according to [2], comprising:
   (a) culturing a reference yeast and a test yeast;
   (b) quantifying the protein in each yeast encoded by the polynucleotide selected from the group consisting of:
      (i) a polynucleotide comprising the nucleotide sequence of SEQ ID NO:1;
      (ii) a polynucleotide encoding a protein consisting of the amino acid sequence of SEQ ID NO:2; and
      (iii) a polynucleotide encoding a protein having an amino acid sequence having 93% or higher identity with the amino acid sequence of SEQ ID NO:2 having catalase activity and an enhanced sulfite-producing capability in yeast; and
   (c) selecting a test yeast having said protein in a larger amount than that in the reference yeast.
[5] Use of a polynucleotide selected from the group consisting of:
   (a) a polynucleotide comprising the nucleotide sequence of SEQ ID NO:1;
   (b) a polynucleotide encoding a protein consisting of the amino acid sequence of SEQ ID NO:2 and
   (c) a polynucleotide encoding a protein having an amino acid sequence having 93% or higher identity with the amino acid sequence of SEQ ID NO:2 having catalase activity
   for the preparation of a yeast with an enhanced sulfite-producing capability.
[6] Use of a polynucleotide selected from the group consisting of:
   (a) a polynucleotide comprising the nucleotide sequence of SEQ ID NO:1;
   (b) a polynucleotide encoding a protein consisting of the amino acid sequence of SEQ ID NO:2; and
   (c) a polynucleotide encoding a protein having an amino acid sequence having 93% or higher identity with the amino acid sequence of SEQ ID NO:2 having catalase activity
   for enhancing a sulfite-producing capability in a yeast.
[7] A method for enhancing a sulfite-producing capability of a yeast comprising the step of increasing the expression of a polynucleotide selected from the group consisting of:
   (a) a polynucleotide comprising the nucleotide sequence of SEQ ID NO: 1;
   (b) a polynucleotide encoding a protein consisting of the amino acid sequence of SEQ ID NO:2; and
   (c) a polynucleotide encoding a protein having an amino acid sequence having 93% or higher identity with the amino acid sequence of SEQ ID NO:2 having catalase activity
   in said yeast.
[8] A method for producing an alcoholic beverage with increased content of sulfite, comprising culturing a yeast into which a vector comprising the polynucleotide selected from the group consisting of:
   (a) a polynucleotide comprising the nucleotide sequence of SEQ ID NO:1;
   (b) a polynucleotide encoding a protein consisting of the amino acid sequence of SEQ ID NO:2;
   (c) a polynucleotide encoding a protein consisting of the amino acid sequence of SEQ ID NO: 4; and
   (d) a polynucleotide encoding a protein having an amino acid sequence having 93% or higher identity with the amino acid sequence of SEQ ID NO:2 having catalase activity,
   is introduced, wherein the sulfite-producing capability is enhanced.
[9] The method of any one of [3], [4], [7] or[ [8] or the use of [5] or [6], wherein the polynucleotide is DNA.
[10] The method of [8], wherein the brewed alcoholic beverage is a malt beverage.
[11] The method of [8], wherein the brewed alcoholic beverage is wine.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract consumption with time upon beer fermentation test. The horizontal axis represents fermentation time while the vertical axis represents apparent extract concentration (w/w%).
Figure 3 shows the expression behavior of non-ScCTT1 gene in yeasts upon beer fermentation test. The horizontal axis represents fermentation time while the vertical axis represents the brightness of detected signal.
Figure 4 shows the cell growth with time upon beer fermentation test using the non-ScCTT1-highly expressed strain. The horizontal axis represents fermentation time while the vertical axis represents optical density at 660 nm (OD660).
Figure 5 shows the extract consumption with time upon beer fermentation test using the non-ScCTT1-highly expressed strain. The horizontal axis represents fermentation time while the vertical axis represents apparent extract concentration (w/w%).
Figure 6 shows sulfite concentration in the fermentation broth (at the completion of fermentation) during beer fermentation test using the non-ScCTT1-highly expressed strain.
Figure 7 shows the cell growth with time upon beer fermentation test. The horizontal axis represents fermentation time while the vertical axis represents optical density at 660 nm (OD660).
Figure 8 shows the extract consumption with time upon beer fermentation test. The horizontal axis represents fermentation time while the vertical axis represents apparent extract concentration (w/w%).
Figure 9 shows the expression behavior of ScCTT1 gene in yeasts upon beer fermentation test. The horizontal axis represents fermentation time while the vertical axis represents the brightness of detected signal.
Figure 10 shows the cell growths with time upon beer fermentation test using the ScCTT1-highly expressed strain. The horizontal axis represents fermentation time while the vertical axis represents optical density at 660 nm (OD660).
Figure 11 shows the extract consumption with time upon beer fermentation test using the ScCTT1-highly expressed strain. The horizontal axis represents fermentation time while the vertical axis represents apparent extract concentration (w/w%).
Figure 12 shows sulfite concentration in the fermentation broth (at the completion of fermentation) during beer fermentation test using the ScCTT1-highly expressed strain.

### BEST MODES FOR CARRYING OUT THE INVENTION

Disclosed is an isolated and identified non-ScCTT1 gene encoding a protein having a catalase activity unique to lager brewing yeast based on the larger brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2. Furthermore, the ScCTT1 gene encoding a protein having a catalase activity unique to lager brewing yeast is disclosed herein. The nucleotide sequence of the gene is represented by SEQ ID NO: 3. Further, an amino acid sequence of a protein encoded by the gene is represented by SEQ ID NO: 4.

### 1. Polynucleotide of the invention

First of all, disclosed is (a) a polynucleotide comprising a polynucleotide of the nucleotide sequence of SEQ ID NO:1 or SEQ ID NO: 3; and (b) a polynucleotide comprising a polynucleotide encoding a protein of the amino acid sequence of SEQ ID NO:2 or SEQ ID NO:4. The polynucleotide can be DNA or RNA.

The target polynucleotide of the present invention is not limited to the polynucleotide encoding a protein having a catalase activity derived from lager brewing yeast and may include other polynucleotides encoding proteins having equivalent functions to said protein. Proteins with equivalent functions include, for example (e) a protein having an amino acid sequence with about 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2 and having a catalase activity. In general, the percentage identity is preferably higher.

The catalase activity can be assessed, for example by, a method of Osorio et al., Archives of Microbiology, 181(3), 231-236 (2004).

### 2. Protein of the present invention

The present invention also provides proteins encoded by any of the polynucleotides (a) to (I) above. A preferred protein of the present invention comprises an amino acid sequence of SEQ ID NO:2 or SEQ ID NO: 4.

### 3. Vector of the invention and yeast transformed with the vector

Also disclosed is a vector comprising the polynucleotide described above. The vector of the present invention is directed to a vector including any of the polynucleotides (DNA) described in (a) to (c) above. Generally, the vector of the present invention comprises an expression cassette including as components (x) a promoter that can transcribe in a yeast cell; (y) a polynucleotide (DNA) described in any of (a) to (I) above that is linked to the promoter in sense or antisense direction, and (z) a signal that functions in the yeast with respect to transcription termination and polyadenylation ofRNA molecule.

A vector introduced in the yeast may be any of a multicopy type (YEp type), a single copy type (YCp type), or a chromosome integration type (YIp type). For example, YEp24 (J. R Broach al., Experimental Manipulation of Gene Expression, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they have no influence on the concentration of constituents in fermentation broth. For example, a promoter of glyceraldehydes 3-phosphate dehydrogenase gene (TDH3), or a promoter of 3-phosphoglycerate kinase gene (PGK 1) may be used These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used.

A lager brewing yeast, for example, *Saccharomyces pastorianus* W34/70, *Saccharomyces carlsbergensis* NCYC453 or NCYC456, or *Saccharomyces cerevisiae* NBRC1951, NBRC1952, NBRC1953 or NBRC1954 may be used herein. In addition, wine yeasts such as wine yeasts #1, 3 and 4 from the Brewing Society of Japan, and sake yeasts such as sake yeast #7 and 9 from the Brewing Society of Japan may also be used but not limited thereto. Lager brewing yeasts such as *Saccharomyces pastorianus* may be used preferably herein.

A yeast transformation method may be a generally used known method. For example, methods that can be used include but not limited to an electroporation method (Meth. Enzym, 194: 182 (1990)), a spheroplast method (Proc. Natl. Acad. Sci. USA, 75: 1929(1978)), a lithium acetate method (J. Bacteriology, 153: 163 (1983)), and methods described in Proc. Natl. Acad. Sci. USA, 75: 1929 (1978), Methods in Yeast Genetics, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali ion metal ion, preferably lithium ion at a concentration of about 1 to 2 M After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in Molecular Cloning 3rd Ed., and Methods in Yeast Genetics, A Laboratory Manual (Cold Spring Harbor Laboratory Press, Cold Spring Harbor, NY).

### 4. Methods of producing alcoholic beverages according to the present invention and alcoholic beverages produced by the method

The vector described above is introduced into a yeast suitable for brewing a target alcoholic product. This yeast can be used to increase content of sulfite of desired alcoholic beverages with superior stability of flavor. In addition, yeasts to be selected by the yeast assessment method described below can also be used. The target alcoholic beverages include, for example, but not limited to beer, sparkling liquor (*happoushu*) such as a beer-taste beverage, wine, sake and the like.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with increased content of sulfite. Thus, alcoholic beverages with superior stability of flavor can be produced using the existing facility without increasing the cost.

### 5. Yeast assessment method of the invention,

The invention relates to a method for assessing a test yeast for its capability of producing sulfite by using a primer or a probe designed based on a nucleotide sequence of a gene having the nucleotide sequence of SEQ ID NO: or SEQ ID NO: 3, and encoding a protein having a catalase activity. General techniques for such assessment method are known and are described in, for example, WO01/040514, Japanese Laid-Open Patent Application No. 8-205900 or the like. This assessment method is described in below.

First, genome of a test yeast is prepared. For this preparation; any known method such as Hereford method or potassium acetate method may be used (e.g., Methods in Yeast Genetics, Cold Spring Harbor Laboratory Press, 130 (1990)). Using a primer or a probe designed based on a nucleotide sequence. (preferably, ORF sequence) of the gene encoding a protein having a catalase activity, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capability of producing sulfite of the yeast as determined by whether the molecular weight of the amplified product is a size that contains the DNA molecule of the specific part. In addition, by analyzing the nucleotide sequence of the amplified product, the capability may be predicted and/or assessed more precisely.

Moreover, in the invention, a test yeast is cultured to measure an expression level of the gene having the nucleotide sequence of SEQ ID NO: 1 or SEQ ID NO: 3 and encoding a protein having a catalase activity to assess the test yeast for its capability of producing sulfite. In measuring an expression level of the gene encoding a protein having a catalase activity, the test yeast is cultured, and then mRNA or a protein resulting from the gene encoding a protein having a catalase activity, is quantified. The quantification of mRNA or protein may be carried out by employing a known technique. For example, mRNA may be quantified, by Northern hybridization or quantitative RT-PCR, while protein may be quantified, for example, by Western blotting (Current Protocols in Molecular Biology, John Wiley & Sons 1994-2003). Further, expression level of the above-identified gene of the test yeast may be projected by measuring sulfite concentration of fermentation broth obtained after fermentation of the test yeast.

Furthermore, test yeasts are cultured and expression levels of the gene having the nucleotide sequence of SEQ ID NO: 1 or SEQ ID NO: 3, and encoding a protein having a catalase activity are measured to select a test yeast with the gene expression level according to the target catalase activity, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the gene having the nucleotide sequence of SEQ ID NO: 1 or SEQ ID NO: 3 and encoding a protein having a catalase activity, is measured in each yeast. By selecting a test yeast with the gene expressed higher than that in the reference yeast, a yeast suitable for brewing alcoholic beverages can be selected.

Alternatively, test yeasts are cultured and a yeast with a higher catalase activity is selected, thereby selecting a yeast suitable for brewing desired alcoholic beverages.

In these cases, the test yeasts or the reference yeast may be, for example, a yeast introduced with the vector of the invention, an artificially mutated yeast or a naturally mutated yeast. The catalase activity can be assessed, for example by, a method of Osorio et al., Archives of Microbiology, 181(3), 231-236 (2004). The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (see, e.g., Yasuji Oshima Ed., Biochemistry Experiments vol. 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (e.g., *S. pastorianus, S. cerevisiae,* and *S*. *carlsbergensis*). A lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954 may be used herein. Further, whisky yeasts such as *Saccharomyces carlsbergensis* NCYC90, wine yeasts such as wine yeasts #1, 3 and 4 from the Brewing Society of Japan; and sake yeasts such as sake yeast #7 and 9 from the Brewing Society of Japan may also be used but not limited thereto.

Lager brewing yeasts such as *Saccharomyces pastorianus* may preferably be used herein. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Gene Encoding Catalase of Lager Brewing Yeast (non-ScCTT1)

A gene encoding a catalase (non-ScCTT1 gene; SEQ ID NO: 1) specific to a lager brewing yeast was found, as a result of a search utilizing the comparison database described in Japanese Patent Application Laid-Open No. 2004-283169. Based on the acquired nucleotide sequence information, primers non-ScCTT1_for (SEQ ID NO: 5) and non-ScCTT1_rv (SEQ ID NO: 6) were designed to amplify the full-length genes, respectively. PCR was carried out using chromosomal DNA of a genome sequencing strain, *Saccharomyces pastorianus* Weihenstephan 34/70 strain (also sometimes referred to as "W34/70 strain"), as a template to obtain DNA fragments including the full-length gene of non-ScCTT1

The thus-obtained non-ScCTT1 gene fragment was inserted into pCR2.1-TOPO vector (manufactured by Invitrogen Corporation) by TA cloning. The nucleotide sequences of non-ScCTT1 gene were analyzed according to Sanger's method (F. Sanger, Science, 214: 1215, 1981) to confirm the nucleotide sequence.

### Example 2: Analysis of Expression of non-ScCTT1 Gene during Beer Fermentation

A beer fermentation test was conducted using a lager brewing yeast, *Saccharomyces pastorianus* 34/70 strain and then mRNA extracted from yeast cells during fermentation was analyzed by a yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8x10⁶ cells/mL |

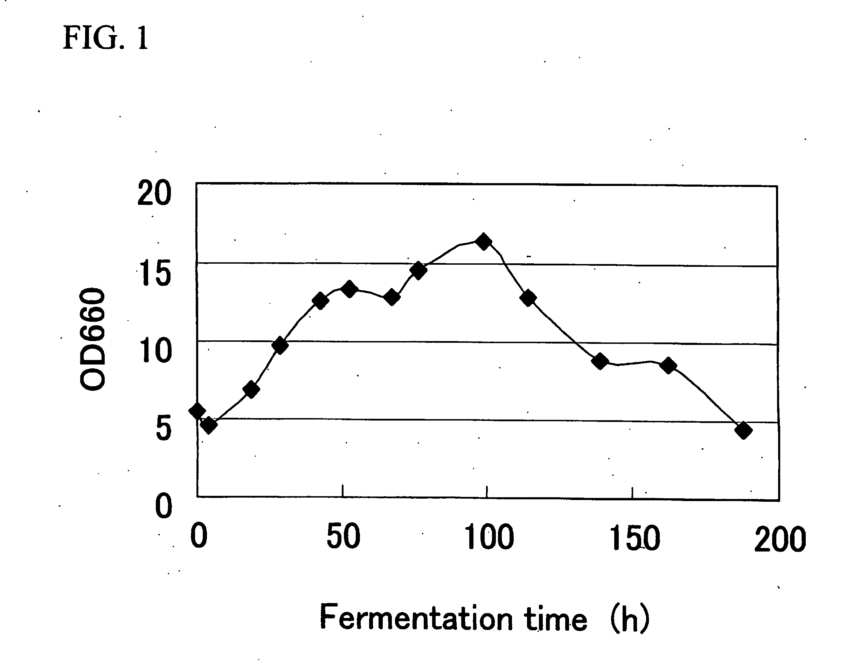

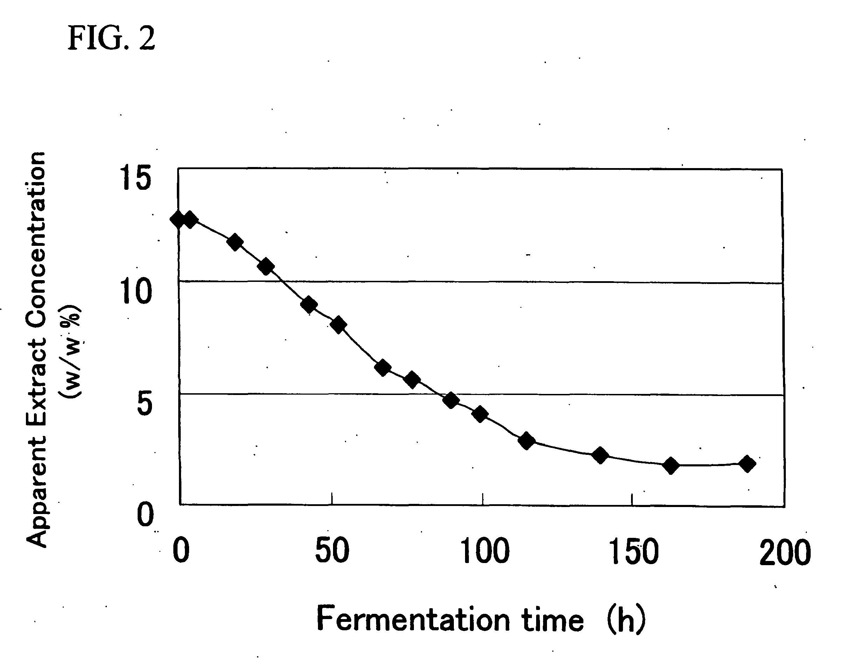

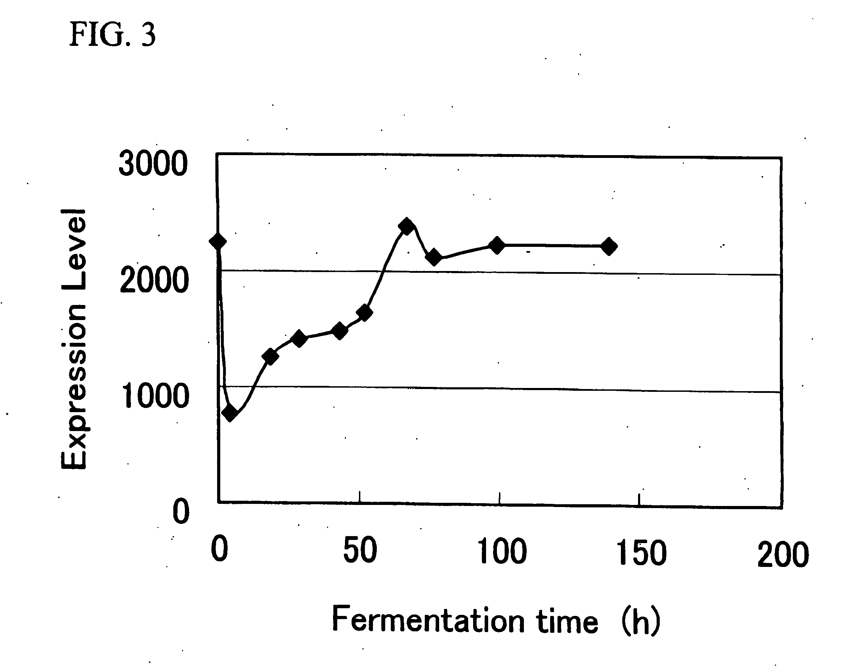

Sampling of fermentation liquid was performed with time, and variation with time of yeast growth amount (Fig. 1) and apparent extract concentration (Fig. 2) was observed. Simultaneously, yeast cells were sampled to prepare mRNA, and the prepared mRNA was labeled with biotin and was hybridized to a beer yeast DNA microarray. The signal was detected using GCOS; GeneChip Operating Software 1.0 (manufactured by Affymetrix Co.). Expression pattern of non-ScCTT1 gene is shown in Figure 3. As a result, it was confirmed that non-ScCTT1 gene was expressed in the general beer fermentation

### Example 3: Preparation of non-ScCTT1 Gene-Highly Expressed Strain

The non-ScCTT1/pCR2.1-TOPO described in Example 1 was digested with restriction enzymes SacI and NotI to prepare a DNA fragment including non-ScCTT1 gene. This fragment was linked to pUP3GLP2 treated with restriction enzymes SacI and NotI, thereby constructing a non-ScCTT1 high expression vector, pUP-nonScCTT1. The yeast expression vector, pUP3GLP2, is a YIp type (chromosome integration type) yeast expression vector having orotidine-5-phosphoric acid decarboxylase gene URA3 at the homologous recombinant site. The introduced gene was highly expressed by the promoter and terminator of glycerylaldehyde-3-phosphoric acid dehydrogenase gene, TDH3. Drug-resistant gene YAP1 as a selective marker for yeast was introduced under the control of the promoter and terminator of galactokinase GAL1, whereby the expression is induced in a culture media comprising galactose. Ampicillin-resistant gene Amp as a selective marker for *E. coli* was also included.

Using the high expression vector prepared by the above method, *Saccharomyces pastorianus* Weihenstephan 164 strain was transformed by the method described in Japanese Patent Application Laid-Open No. 07-303475. A cerulenin-resistant strain was selected in a YPGal plate medium (1% yeast extract, 2% polypeptone, 2% galadose, 2% agar) containing 1.0 mg/L of cerulenin.

### Example 4: Analysis of Amount of Sulfite Produced during Beer Fermentation

The parent strain and non-ScCTT1-highly expressed strain obtained in Example 3 were used to carry out fermentation test under the following conditions.

| | |
|---|---|
| Wort extract concentration | 12.78% |
| Wort content | 2 L |
| Wort dissolved oxygen concentration | approximately 8 ppm |
| Fermentation temperature | 15°C, constant |
| Yeast pitching rate | 10.5 g wet yeast cells/2L Wort |

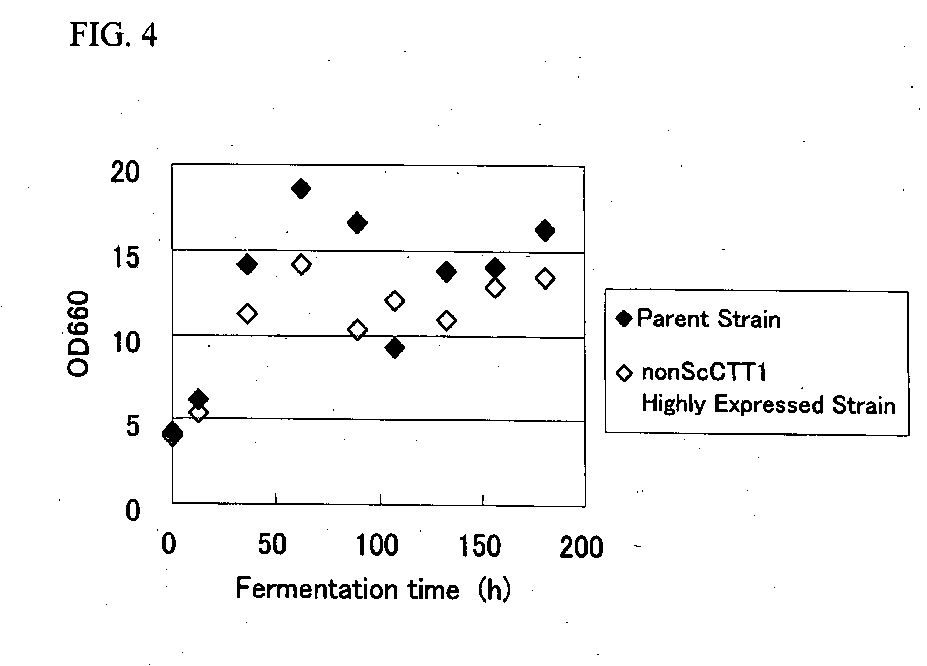

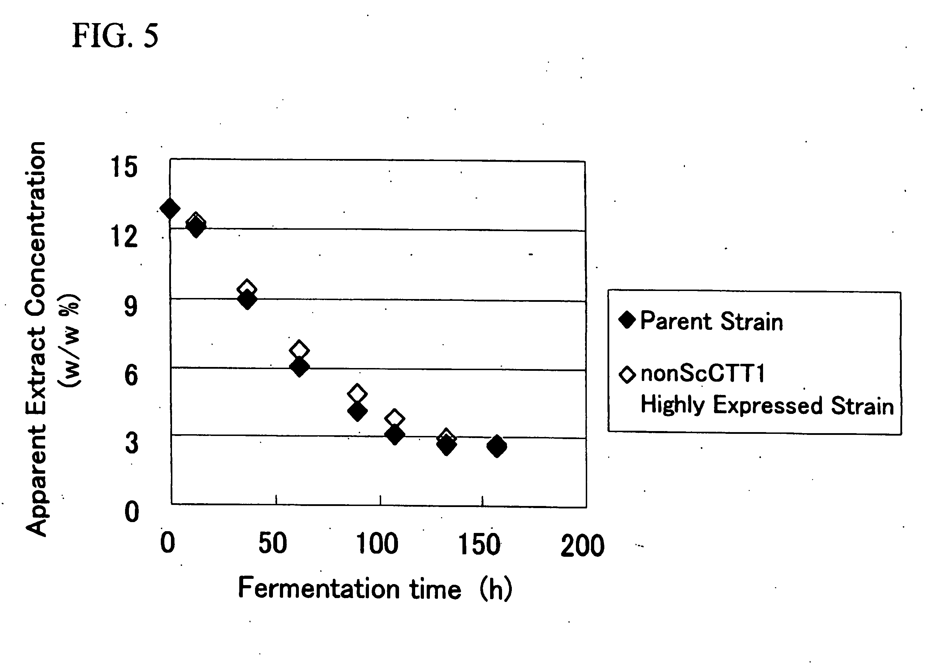

The fermentation broth was sampled with time to observe the cell growth (OD660) (Fig. 4) and extract consumption with time (Fig. 5). Quantification of sulfite concentration at completion of fermentation was carried out by collecting sulfite into hydrogen peroxide aqueous solution by distillation under acidic condition, and titration with alkali (Revised BCOJ Beer Analysis Method by the Brewing Society of Japan).

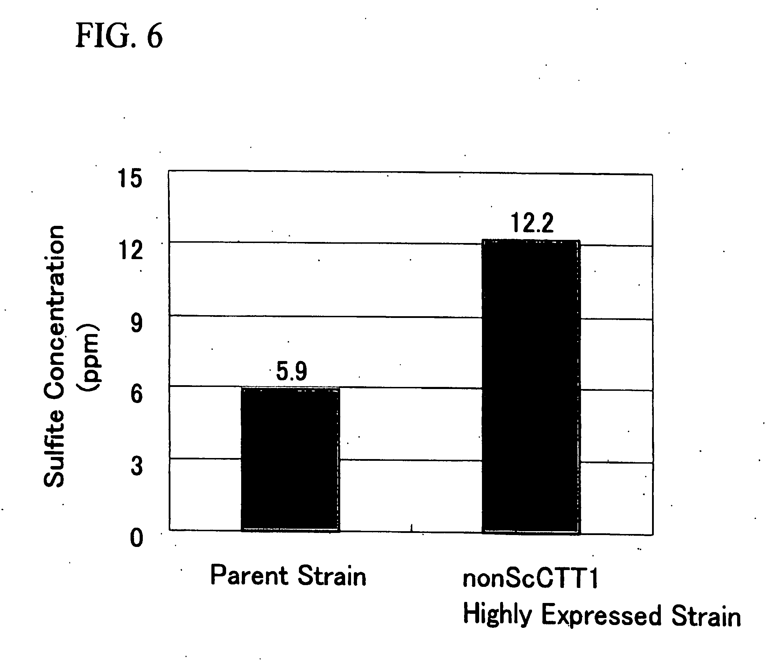

As shown in Fig. 6, the non-ScCTT1-highly expressed strain produced sulfite approximately 2.1 times greater than the parent strain. In addition, significant differences were not observed between the parent strain and the highly expressed strain in cell growth and extract consumption in this testing.

### Example 5: Cloning of Gene Encoding Catalase (ScCTT1)

A gene encoding a catalase (ScCTT1 gene; SEQ ID NO: 3) specific to a lager brewing yeast was found, as a result of a search utilizing the comparison database described in Japanese Patent Application Laid-Open No. 2004-283169. Based on the acquired nucleotide sequence information, primers ScCTT1_for (SEQ ID NO: 7) and ScCTT1_rv (SEQ ID NO: 8) were designed to amplify the full-length genes, respectively. PCR was carried out using chromosomal DNA of a genome sequencing strain, *Saccharomyces pastorianus* Weihenstephan 34/70 strain, as a template to obtain DNA fragments including the full-length gene of ScCTT1.

The thus-obtained ScCTT1 gene fragment was inserted into pCR2.1-TOPO vector (manufactured by Invitrogen Corporation) by TA cloning. The nucleotide sequences of ScCTT1 gene were analyzed according to Sanger's method (F. Sanger, Science, 214: 1215, 1981) to confirm the nucleotide sequence.

### Example 6: Analysis of Expression of ScCTT1 Gene during Beer Fermentation

A beer fermentation test was conducted using a lager brewing yeast, *Saccharomyces pastorianus* 34/70 strain and then mRNA extracted from yeast cells during fermentation was analyzed by a yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12. 8x 10⁶ cells/mL |

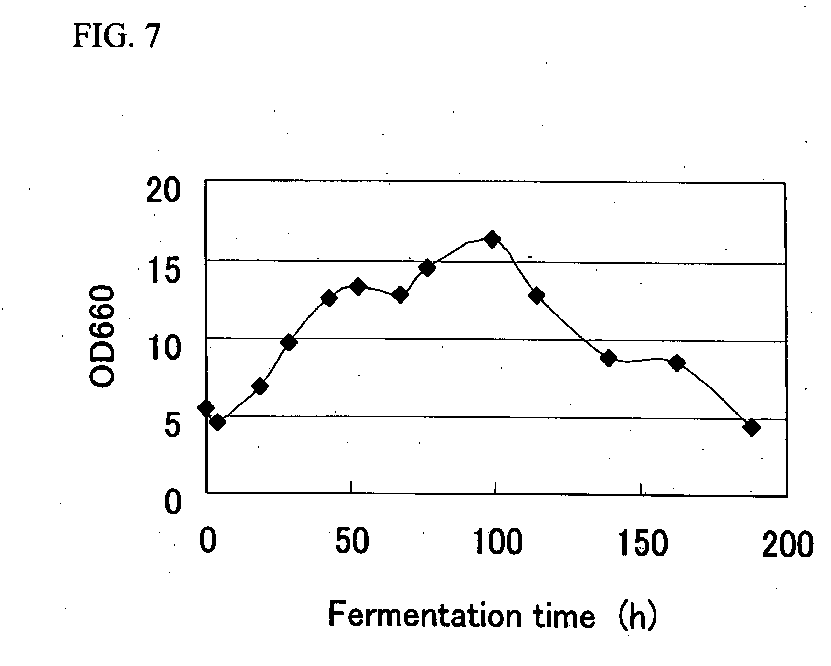

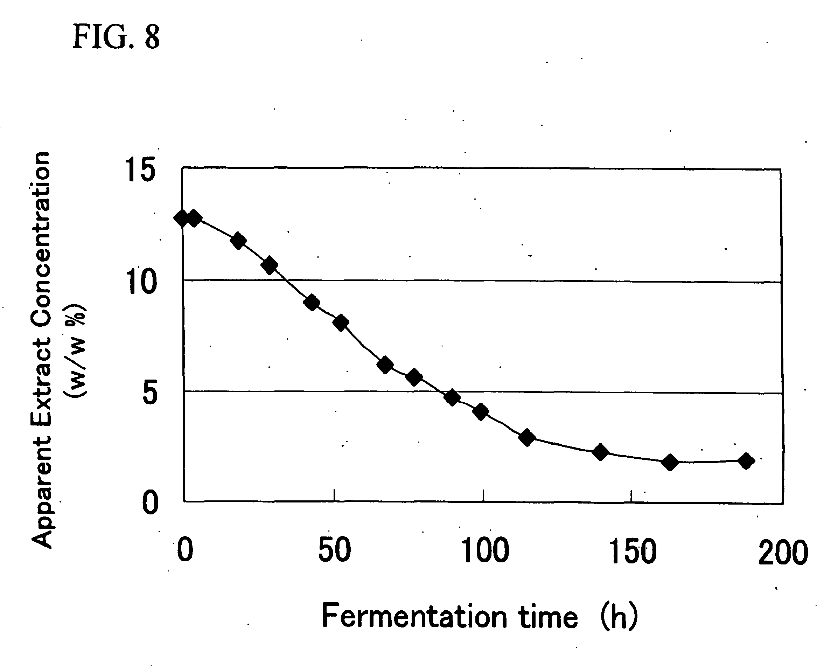

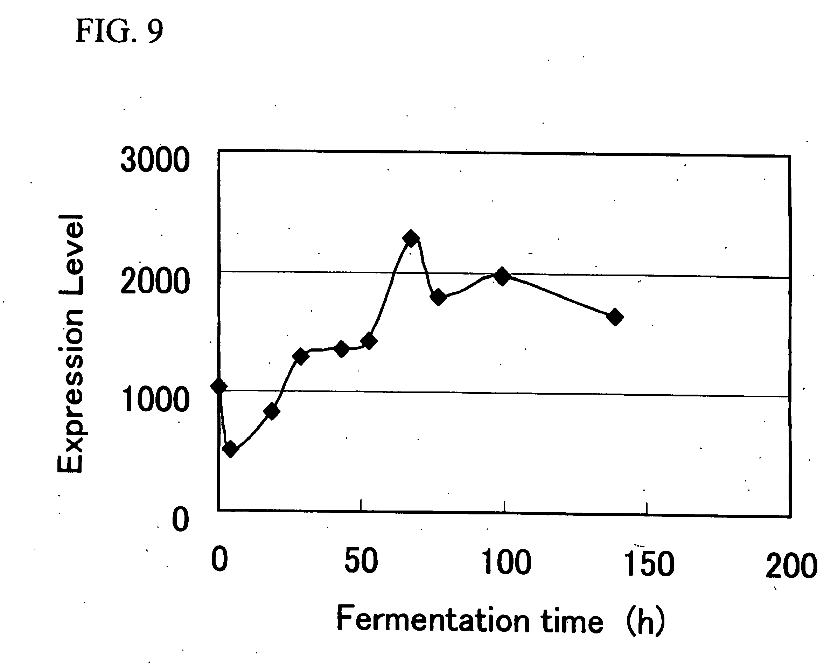

Sampling of fermentation liquid was performed with time, and variation with time of yeast growth amount (Fig. 7) and apparent extract concentration (Fig. 8) was observed. Simultaneously, yeast cells were sampled to prepare mRNA, and the prepared mRNA was labeled with biotin and was hybridized to a beer yeast DNA microarray. The signal was detected using GCOS; GeneChip Operating Software 1.0 (manufactured by Affymetrix Co.). Expression pattern of ScCTT1 gene is shown in Figure 9. As a result, it was confirmed that ScCTT1 gene was expressed in the general beer fermentation.

### Example 7: Preparation of ScCTT1-Highly Expressed Strain

The ScCTT1/pCR2.1-TOPO described in Example 5 was digested with restriction enzymes SacI and NotI to prepare a DNA fragment including ScCTT1 gene. This fragment was linked to pUP3GLP2 treated with restriction enzymes SacI and NotI, thereby constructing a ScCTT1 high expression vector, pUP-ScCTT1.

Using the high expression vector prepared by the above method, *Saccharomyces pasteurianus* Weihenstephaner 164 strain was transformed by the methods described in Japanese Patent Application Laid-open No. H7-303475. A cerulenin-resistant strain was selected in a YPGal plate medium (1% yeast extract, 2% polypeptone, 2% galactose, 2% agar) containing 1.0 mg/L of cerulenin.

### Example 8: Analysis of Amount or Sulfite Produced during Beer Fermentation

The parent strain and ScCTT1-highly expressed strain obtained in Example 7, are used to carry out fermentation test under the following conditions.

| | |
|---|---|
| Wort extract concentration | 12.78% |
| Wort content | 2 L |
| Wort dissolved oxygen concentration | approximately 8 ppm |
| Fermentation temperature | 15°C, constant |
| Yeast pitching rate | 10.5 g wet yeast cells/2L Wort |

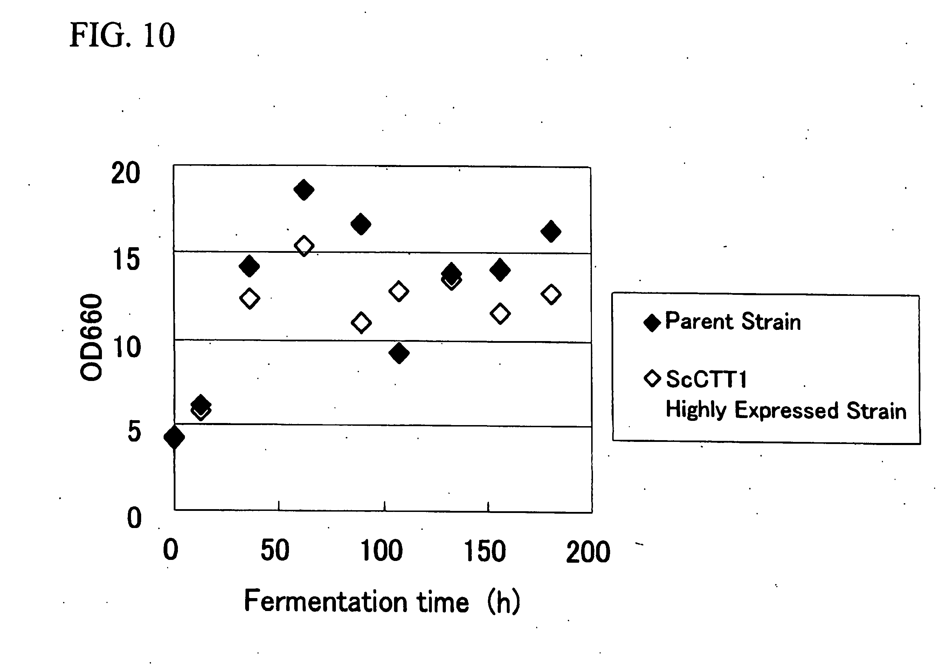

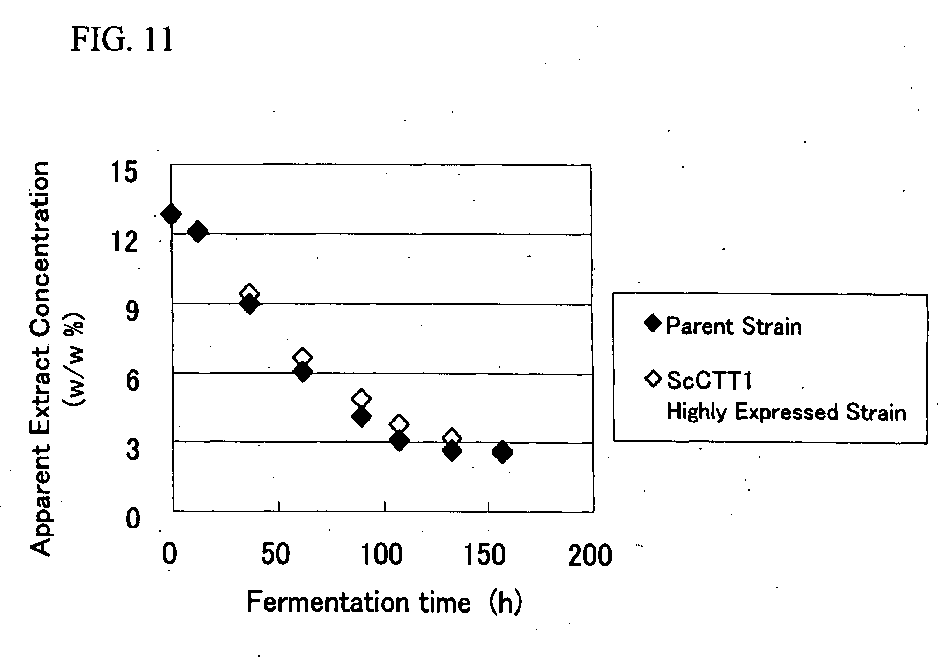

The fermentation broth was sampled with time to observe the cell growth (OD660) (Fig. 10) and extract consumption with time (Fig. 11). Quantification of sulfite concentration at completion of fermentation was carried out by collecting sulfite into hydrogen peroxide aqueous solution by distillation under acidic condition, and titration with alkali (Revised BCOJ Beer Analysis Method by the Brewing Society of Japan).

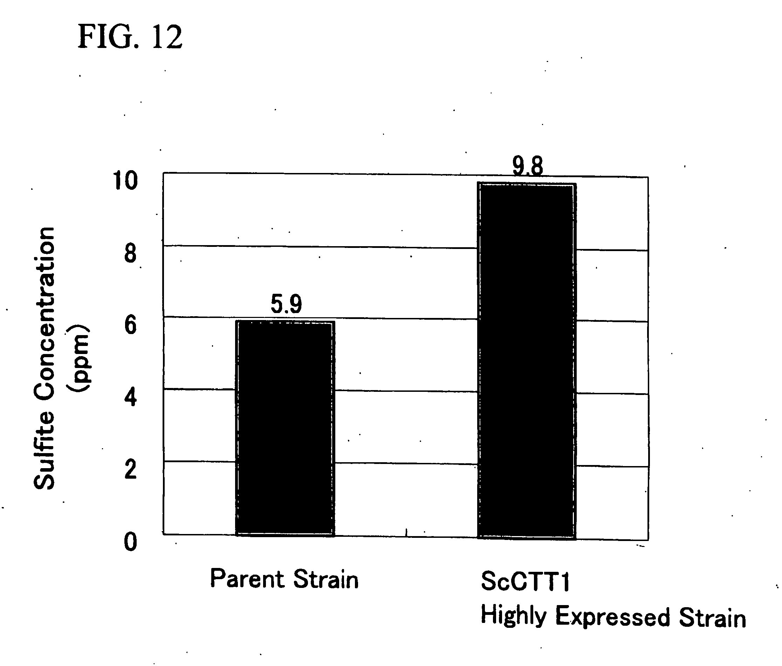

As shown in Fig. 12, the ScCTT1-highly expressed strain produced sulfite approximately 1.7 times greater than the parent strain. In addition, significant differences were not observed between the parent strain and the highly expressed strain in cell growth and extract consumption in this testing.

### INDUSTRIAL APPLICABILITY

According to the method for producing alcoholic beverages of the present invention, because of increase in amount of sulfite which has an anti-oxidative effect in products, alcoholic beverages with superior flavor stability and longer shelf life can be produced.

## Claims

1. A method for assessing a test yeast for its sulfite-producing capability, comprising:
(a) culturing a test yeast; and
(b) measuring the expression level of a gene encoding a protein having catalase activity said gene having the nucleotide sequence of SEQ ID NO: 1 or SEQ ID NO: 3 by using a primer or a probe designed based on the nucleotide sequence of SEQ ID NO: 1 or SEQ ID NO: 3.

2. A method for selecting a yeast, comprising:
(a) culturing a test yeast;
(b) quantifying the protein encoded by the polynucleotide selected from the group consisting of:
(i) a polynucleotide comprising the nucleotide sequence of SEQ ID NO: 1;
(ii) a polynucleotide encoding a protein consisting of the amino acid sequence of SEQ ID NO:2; and
(iii) a polynucleotide encoding a protein having an amino acid sequence having 93% or higher identity with the amino acid sequence of SEQ ID NO:2 having catalase activity and an enhanced sulfite-producing capability in yeast or measuring the expression level of a gene encoding a protein having catalase activity said gene having the nucleotide sequence of SEQ ID NO: 1 or SEQ ID NO: 3; and
(c) selecting a test yeast having desired amount of said protein or desired expression level of said gene in view of a desired sulfite-producing capability.

3. The method for selecting a yeast according to claim 2, comprising:
(a) culturing a reference yeast and a test yeast;
(b) measuring the expression level of a gene encoding a protein having catalase activity in each yeast, said gene having the nucleotide sequence of SEQ ID NO: 1 or SEQ ID NO: 3; and
(c) selecting a test yeast having the gene expressed higher than that in the reference yeast.

4. The method for selecting a yeast according to claim 2, comprising:
(a) culturing a reference yeast and a test yeast;
(b) quantifying the protein in each yeast encoded by the polynucleotide selected from the group consisting of:
(i) a polynucleotide comprising the nucleotide sequence of SEQ ID NO: 1;
(ii) a polynucleotide encoding a protein consisting of the amino acid sequence of SEQ ID NO:2; and
(iii) a polynucleotide encoding a protein having an amino acid sequence having 93% or higher identity with the amino acid sequence of SEQ ID NO:2 having catalase activity and an enhanced sulfite-producing capability in yeast; and
(c) selecting a test yeast having said protein in a larger amount than that in the reference yeast.

5. Use of a polynucleotide selected from the group consisting of:
(a) a polynucleotide comprising the nucleotide sequence of SEQ ID NO:1;
(b) a polynucleotide encoding a protein consisting of the amino acid sequence of SEQ ID NO:2; and
(c) a polynucleotide encoding a protein having an amino acid sequence having 93% or higher identity with the amino acid sequence of SEQ ID NO:2 having catalase activity
for the preparation of a yeast with an enhanced sulfite-producing capability.

6. Use of a polynucleotide selected from the group consisting of:
(a) a polynucleotide comprising the nucleotide sequence of SEQ ID NO:1;
(b) a polynucleotide encoding a protein consisting of the amino acid sequence of SEQ ID NO:2; and
(c) a polynucleotide encoding a protein having an amino acid sequence having 93% or higher identity with the amino acid sequence of SEQ ID NO:2 having catalase activity
for enhancing a sulfite-producing capability in a yeast.

7. A method for enhancing a sulfite-producing capability of a yeast comprising the step of increasing the expression of a polynucleotide selected from the group consisting of:
(a) a polynucleotide comprising the nucleotide sequence of SEQ ID NO:1;
(b) a polynucleotide encoding a protein consisting of the amino acid sequence of SEQ ID NO:2; and
(c) a polynucleotide encoding a protein having an amino acid sequence having 93% or higher identity with the amino acid sequence of SEQ ID NO:2 having catalase activity
in said yeast.

8. A method for producing an alcoholic beverage with increased content of sulfite, comprising culturing a yeast into which a vector comprising the polynucleotide selected from the group consisting of:
(a) a polynucleotide comprising the nucleotide sequence of SEQ ID NO:1;
(b) a polynucleotide encoding a protein consisting of the amino acid sequence of SEQ ID NO:2;
(c) a polynucleotide encoding a protein consisting of the amino acid sequence of SEQ ID NO: 4; and
(d) a polynucleotide encoding a protein having an amino acid sequence having 93% or higher identity with the amino acid sequence of SEQ ID NO:2 having catalase activity,
is introduced, wherein the sulfite-producing capability is enhanced.

9. The method of any one of claims 3, 4, 7 or 8 or the use of claim 5 or 6, wherein the polynucleotide is DNA.

10. The method of claim 8, wherein the brewed alcoholic beverage is a malt beverage.

11. The method of claim 8, wherein the brewed alcoholic beverage is wine.

## Patentansprüche

1. Verfahren zur Bewertung einer Testhefe bezüglich ihrer Sulfit-produzierenden Fähigkeit, umfassend:
(a) Züchten einer Testhefe; und
(b) Messen des Expressionsspiegels eines Gens, das ein Protein mit Katalase-Aktivität codiert, wobei das Gen die Nucleotidsequenz von SEQ ID NO:1 oder SEQ ID NO:3 hat, unter Verwendung eines Primers oder einer Sonde, welche(r) anhand der Nucleotidsequenz von SEQ ID NO:1 oder SEQ ID NO:3 entworfen wurde(n).

2. Verfahren zum Auswählen einer Hefe, umfassend:
(a) Züchten einer Testhefe;
(b) Quantifizieren des Proteins, das durch das Polynucleotid codiert wird, das ausgewählt ist aus der Gruppe bestehend aus:
(i) einem Polynucleotid, umfassend die Nucleotidsequenz von SEQ ID NO:1;
(ii) einem Polynucleotid, das ein Protein codiert, das aus der Aminosäuresequenz von SEQ ID NO:2 besteht; und
(iii) einem Polynucleotid, das ein Protein mit einer Aminosäuresequenz codiert, die eine 93%-ige oder höhere Identität mit der Aminosäuresequenz von SEQ ID NO:2 hat, das Katalase-Aktivität und eine verstärkte Sulfit-produzierende Fähigkeit in Hefe hat, oder Messen des Expressionsspiegels eines Gens, das ein Protein mit Katalase-Aktivität codiert, wobei das Gen die Nucleotidsequenz von SEQ ID NO:1 oder SEQ ID NO:3 hat; und
(c) Auswählen einer Testhefe, welche die gewünschte Menge des Proteins oder den gewünschten Expressionsspiegel des Gens im Hinblick auf die gewünschte Sulfit-produzierende Fähigkeit hat.

3. Verfahren zum Auswählen einer Hefe gemäß Anspruch 2, umfassend:
(a) Züchten einer Referenzhefe und einer Testhefe;
(b) Messen des Expressionsspiegels eines Gens, das ein Protein mit Katalase-Aktivität codiert, in jeder Hefe, wobei das Gen die Nucleotidsequenz von SEQ ID NO:1 oder SEQ ID NO:3 hat; und
(c) Auswählen einer Testhefe, welche das Gen stärker als das in der Referenzhefe exprimiert.

4. Verfahren zum Auswählen einer Hefe gemäß Anspruch 2, umfassend:
(a) Züchten einer Referenzhefe und einer Testhefe;
(b) Quantifizieren des Proteins in jeder Hefe, das durch das Polynucleotid codiert wird, das ausgewählt ist aus der Gruppe bestehend aus:
(i) einem Polynucleotid, umfassend die Nucleotidsequenz von SEQ ID NO:1;
(ii) einem Polynucleotid, das ein Protein codiert, das aus der Aminosäuresequenz von SEQ ID NO:2 besteht; und
(iii) einem Polynucleotid, das ein Protein mit einer Aminosäuresequenz codiert, die eine 93%-ige oder höhere Identität mit der Aminosäuresequenz von SEQ ID NO:2 hat, das Katalase-Aktivität und eine verstärkte Sulfit-produzierende Fähigkeit in Hefe hat; und
(c) Auswählen einer Testhefe, welche das Protein in einer größeren Menge als die in der Referenzhefe hat.

5. Verwendung eines Polynucleotids ausgewählt aus der Gruppe bestehend aus:
(a) einem Polynucleotid, umfassend die Nucleotidsequenz von SEQ ID NO:1;
(b) einem Polynucleotid, das ein Protein codiert, das aus der Aminosäuresequenz von SEQ ID NO:2 besteht; und
(c) einem Polynucleotid, das ein Protein mit einer Aminosäuresequenz codiert, die eine 93%-ige oder höhere Identität mit der Aminosäuresequenz von SEQ ID NO:2 hat, das Katalase-Aktivität hat,
zur Herstellung einer Hefe mit einer verstärkten Sulfit-produzierenden Fähigkeit.

6. Verwendung eines Polynucleotids ausgewählt aus der Gruppe bestehend aus:
(a) einem Polynucleotid, umfassend die Nucleotidsequenz von SEQ ID NO:1;
(b) einem Polynucleotid, das ein Protein codiert, das aus der Aminosäuresequenz von SEQ ID NO:2 besteht; und
(c) einem Polynucleotid, das ein Protein mit einer Aminosäuresequenz codiert, die eine 93%-ige oder höhere Identität mit der Aminosäuresequenz von SEQ ID NO:2 hat, das Katalase-Aktivität hat,
zur Verstärkung der Sulfit-produzierenden Fähigkeit in einer Hefe.

7. Verfahren zur Verstärkung einer Sulfit-produzierenden Fähigkeit einer Hefe, umfassend den Schritt der Steigerung der Expression eines Polynucleotids ausgewählt aus der Gruppe bestehend aus:
(a) einem Polynucleotid, umfassend die Nucleotidsequenz von SEQ ID NO:1;
(b) einem Polynucleotid, das ein Protein codiert, das aus der Aminosäuresequenz von SEQ ID NO:2 besteht; und
(c) einem Polynucleotid, das ein Protein mit einer Aminosäuresequenz codiert, die eine 93%-ige oder höhere Identität mit der Aminosäuresequenz von SEQ ID NO:2 hat, das Katalase-Aktivität hat,
in der Hefe.

8. Verfahren zum Herstellen eines alkoholischen Getränks mit erhöhtem Sulfitgehalt, umfassend das Züchten einer Hefe, in die ein Vektor eingeführt wurde, umfassend das Polynucleotid ausgewählt aus der Gruppe bestehend aus:
(a) einem Polynucleotid, umfassend die Nucleotidsequenz von SEQ ID NO:1;
(b) einem Polynucleotid, das ein Protein codiert, das aus der Aminosäuresequenz von SEQ ID NO:2 besteht;
(c) einem Polynucleotid, das ein Protein codiert, das aus der Aminosäuresequenz von SEQ ID NO:4 besteht; und
(d) einem Polynucleotid, das ein Protein mit einer Aminosäuresequenz codiert, die eine 93%-ige oder höhere Identität mit der Aminosäuresequenz von SEQ ID NO:2 hat, das Katalase-Aktivität hat,
wobei die Sulfit-produzierende Fähigkeit verstärkt ist.

9. Verfahren nach einem der Ansprüche 3, 4, 7 oder 8 oder die Verwendung nach Anspruch 5 oder 6, wobei das Polynucleotid DNA ist.

10. Verfahren nach Anspruch 8, wobei das gebraute alkoholische Getränk ein Malzgetränk ist.

11. Verfahren nach Anspruch 8, wobei das gebraute alkoholische Getränk Wein ist.

## Revendications

1. Procédé d'évaluation d'une levure à tester pour sa capacité de production de sulfites, comprenant :
(a) la culture d'une levure à tester ; et
(b) la mesure du taux d'expression d'un gène codant pour une protéine ayant une activité catalase, ledit gène ayant la séquence nucléotidique de SEQ ID NO : 1 ou SEQ ID NO : 3 en utilisant une amorce ou une sonde conçue en se basant sur la séquence nucléotidique de SEQ ID NO : 1 ou SEQ ID NO : 3.

2. Procédé de sélection d'une levure, comprenant :
(a) la culture d'une levure à tester ;
(b) la quantification de la protéine codée par le polynucléotide choisi dans le groupe constitué par :
(i) un polynucléotide comprenant la séquence nucléotidique de SEQ ID NO : 1 ;
(ii) un polynucléotide codant pour une protéine constituée de la séquence d'acides aminés de SEQ ID NO : 2 ; et
(iii) un polynucléotide codant pour une protéine ayant une séquence d'acides aminés présentant 93 % d'identité ou plus avec la séquence d'acides aminés de SEQ ID NO : 2 ayant une activité catalase et une capacité améliorée de production de sulfites dans la levure ou la mesure du taux d'expression d'un gène codant pour une protéine ayant une activité catalase, ledit gène ayant la séquence nucléotidique de SEQ ID NO : 1 ou SEQ ID NO : 3 ; et
(c) la sélection d'une levure à tester ayant la quantité souhaitée de ladite protéine ou le taux d'expression souhaité dudit gène au vu d'une capacité souhaitée de production de sulfites.

3. Procédé de sélection d'une levure selon la revendication 2, comprenant :
(a) la culture d'une levure de référence et d'une levure à tester ;
(b) la mesure du taux d'expression d'un gène codant pour une protéine ayant une activité catalase dans chaque levure, ledit gène ayant la séquence nucléotidique de SEQ ID NO : 1 ou SEQ ID NO : 3 ; et
(c) la sélection d'une levure à tester ayant le gène exprimé de façon plus importante que dans la levure de référence.

4. Procédé de sélection d'une levure selon la revendication 2, comprenant :
(a) la culture d'une levure de référence et d'une levure à tester ;
(b) la quantification de la protéine dans chaque levure codée par le polynucléotide choisi dans le groupe constitué par :
(i) un polynucléotide comprenant la séquence nucléotidique de SEQ ID NO : 1 ;
(ii) un polynucléotide codant pour une protéine constituée de la séquence d'acides aminés de SEQ ID NO : 2 ; et
(iii) un polynucléotide codant pour une protéine ayant une séquence d'acides aminés présentant 93 % d'identité ou plus avec la séquence d'acides aminés de SEQ ID NO : 2 ayant une activité catalase et une capacité améliorée de production de sulfites dans la levure ; et
(c) la sélection d'une levure à tester ayant ladite protéine dans une quantité supérieure à celle dans la levure de référence.

5. Utilisation d'un polynucléotide choisi dans le groupe constitué par :
(a) un polynucléotide comprenant la séquence nucléotidique de SEQ ID NO : 1 ;
(b) un polynucléotide codant pour une protéine constituée de la séquence d'acides aminés de SEQ ID NO : 2 ; et
(c) un polynucléotide codant pour une protéine ayant une séquence d'acides aminés présentant 93 % d'identité ou plus avec la séquence d'acides aminés de SEQ ID NO : 2 ayant une activité catalase
pour la préparation d'une levure présentant une capacité de production de sulfites améliorée.

6. Utilisation d'un polynucléotide choisi dans le groupe constitué par :
(a) un polynucléotide comprenant la séquence nucléotidique de SEQ ID NO : 1 ;
(b) un polynucléotide codant pour une protéine constituée de la séquence d'acides aminés de SEQ ID NO : 2 ; et
(c) un polynucléotide codant pour une protéine ayant une séquence d'acides aminés présentant 93 % d'identité ou plus avec la séquence d'acides aminés de SEQ ID NO : 2 ayant une activité catalase
pour améliorer une capacité de production de sulfites dans une levure.

7. Procédé d'amélioration d'une capacité de production de sulfites d'une levure comprenant l'étape d'augmentation de l'expression d'un polynucléotide choisi dans le groupe constitué par :
(a) un polynucléotide comprenant la séquence nucléotidique de SEQ ID NO : 1 ;
(b) un polynucléotide codant pour une protéine constituée de la séquence d'acides aminés de SEQ ID NO : 2 ; et
(c) un polynucléotide codant pour une protéine ayant une séquence d'acides aminés présentant 93 % d'identité ou plus avec la séquence d'acides aminés de SEQ ID NO : 2 possédant une activité catalase
dans ladite levure.

8. Procédé de production d'une boisson alcoolisée présentant une teneur accrue de sulfites, comprenant la culture d'une levure dans laquelle un vecteur comprenant le polynucléotide choisi dans le groupe constitué par :
(a) un polynucléotide comprenant la séquence nucléotidique de SEQ ID NO : 1 ;
(b) un polynucléotide codant pour une protéine constituée de la séquence d'acides aminés de SEQ ID NO : 2 ;
(c) un polynucléotide codant pour une protéine constituée de la séquence d'acides aminés de SEQ ID NO : 4 ; et
(d) un polynucléotide codant pour une protéine ayant une séquence d'acides aminés présentant 93 % d'identité ou plus avec la séquence d'acides aminés de SEQ ID NO : 2 ayant une activité catalase,
est introduit, où la capacité de production de sulfites est améliorée.

9. Procédé selon l'une quelconque des revendications 3, 4, 7 ou 8 ou utilisation selon la revendication 5 ou 6, dans lequel le polynucléotide est de l'ADN.

10. Procédé selon la revendication 8, dans lequel la boisson alcoolisée brassée est une boisson de malt.

11. Procédé selon la revendication 8, dans lequel la boisson alcoolisée brassée est du vin.
